# EUROPEAN PATENT APPLICATION

(11) **EP 2 209 019 A1**
(43) Date of publication of application: **21.07.2010**
(21) Application number: 10150041.1
(22) Date of filing: 04.01.2010
(51) Int. Cl.: G01S 7/52, B06B 1/02

(54) **Power control of transmit pulses in an ultrasound system**

(30) Priority: 16.01.2009 KR 20090003548
(71) Applicant: MEDISON CO., LTD., Kangwon-do 250-870 (KR)
(72) Inventor: Kim, Jin, 135-851, Seoul (KR)
(74) Representative: Schmid, Wolfgang

(57) **Abstract**

An embodiment for controlling a power of transmit pulses in an ultrasound system is disclosed. An ultrasound probe contains a plurality of transducer elements for reciprocal conversion between electrical signals and ultrasound signals. A transmitter includes a plurality of pulse generators. Each of the pulse generators is connected to the respective transducer element through a channel to generate a transmit (Tx) pulse at a transmit (Tx) frequency in synchronization with a Tx control clock. Each of the pulse generators has a plurality of pulsers operating at different voltages in response to pulser control signals.

## Description

### CROSS-REFERENCE TO RELATED APPLICATIONS

The present application claims priority from Korean Patent Application No. 10-2009-003548 filed on January 16, 2009, the entire subject matter of which is incorporated herein by reference.

### TECHNICAL FIELD

The present disclosure relates to ultrasound systems, and more particularly to the power control of transmit (Tx) pulses in a pulse generator of an ultrasound system.

### BACKGROUND

An ultrasound system has become an important and popular diagnostic tool since it has a wide range of applications. Specifically, due to its non-invasive and non-destructive nature, the ultrasound diagnostic system has been extensively used in the medical profession. Modem high-performance ultrasound diagnostic systems and techniques are commonly used to produce two or three-dimensional diagnostic images of internal features of an object (e.g., human organs).

Generally, the ultrasound system transmits ultrasound signals into a target object and receives echo signals reflected from the target object. The ultrasound system may form two or three-dimensional ultrasound images based on the received echo signals. The ultrasound system includes an ultrasound probe for transmitting and receiving ultrasound signals. The ultrasound probe may contain a plurality of transducer elements for reciprocal conversion between ultrasound signals and electrical signals. Also, the ultrasound system may include a plurality of pulse generators to generate Tx pulses. Each of the pulse generators may include a single pulser operating in response to a pulser control signal. The pulse generator may generate the Tx pulses at a transmit (Tx) frequency in synchronization with a transmit (Tx) control clock. The Tx pulses are transmitted to the transducer elements through channels. The transducer elements may be driven in response to the Tx pulses to thereby produce an ultrasound transmit beam.

The ultrasound transmit beam transmitted from the ultrasound probe may include a side lobe or a grating lobe, which may reduce the quality of an ultrasound image. Thus, it is required to reduce the side lobe or the grating lobe from the ultrasound transmit beam. Recently, apodization has been adopted to reduce the side lobe or the grating lobe from an ultrasound transmit beam. The apodization may be performed by controlling the power or waveform of the Tx pulses. The power of the Tx pulses may be controlled through the pulse width modulation (PWM) upon the pulser control signal. Weighting steps to be obtained through the PWM of the pulser control signal may be determined according to the Tx control clock and the Tx frequency. For example, if the frequency of the Tx control clock is increased and the Tx frequency is decreased, then higher resolution of the weighting steps may be expected. On the other hand, if the frequency of the Tx control clock is decreased and the Tx frequency is increased, then lower resolution of the weighting steps may be expected. That is, in order to obtain higher resolution of the weighting steps, the frequency of the Tx control clock should be increased. However, it is difficult to increase the frequency of the Tx control clock due to hardware limitation of the ultrasound system.

### SUMMARY

An embodiment of controlling the power of the transmit pulses in an ultrasound system is disclosed herein. In one embodiment, by way of non-limiting example, an ultrasound system may include: an ultrasound probe containing a plurality of transducer elements for reciprocal conversion between electrical signal and ultrasound signal; and a transmitter including a plurality of pulse generators each connected to the respective transducer element through a channel to generate a transmit (Tx) pulse at a transmit (Tx) frequency in synchronization with a Tx control clock, each of the pulse generators having a plurality of pulsers operating at different voltages in response to pulser control signals.

The Summary is provided to introduce a selection of concepts in a simplified form that are further described below in the Detailed Description. This Summary is not intended to identify key or essential features of the claimed subject matter, nor is it intended to be used in determining the scope of the claimed subject matter.

### BRIEF DESCRIPTION OF THE DRAWINGS

FIG. 1 is a block diagram showing an illustrative embodiment of an ultrasound system.
FIG. 2 is a schematic diagram showing transducer elements, scan lines and a coordinate system.
FIG. 3 is a block diagram showing an illustrative embodiment of the transmitter.
FIG. 4 is a schematic diagram showing an illustrative embodiment of a pulser generator.
FIG. 5A is a schematic diagram showing weights applied to a single pulser for a transmit control clock of 80 MHz and a transmit frequency of 5 MHz.
FIG. 5B is a schematic diagram showing weights applied to dual pulsers for a transmit control clock of 80 MHz and a transmit frequency of 5 MHz.
FIG. 6A is a schematic diagram showing weights applied to a single pulser for a transmit control clock of 80 MHz and a transmit frequency of 10 MHz.
FIG. 6B is a schematic diagram showing weights applied to dual pulsers for a transmit control clock of 80 MHz and a transmit frequency of 10 MHz.
FIG. 7 is a schematic diagram showing an illustrative embodiment of a pulser generator including more than two pulsers.
FIG. 8 is a schematic diagram showing an example of a multi-level transmit waveform.

### DETAILED DESCRIPTION

A detailed description may be provided with reference to the accompanying drawings. One of ordinary skill in the art may realize that the following description is illustrative only and is not in any way limiting. Other embodiments of the present invention may readily suggest themselves to such skilled persons having the benefit of this disclosure.

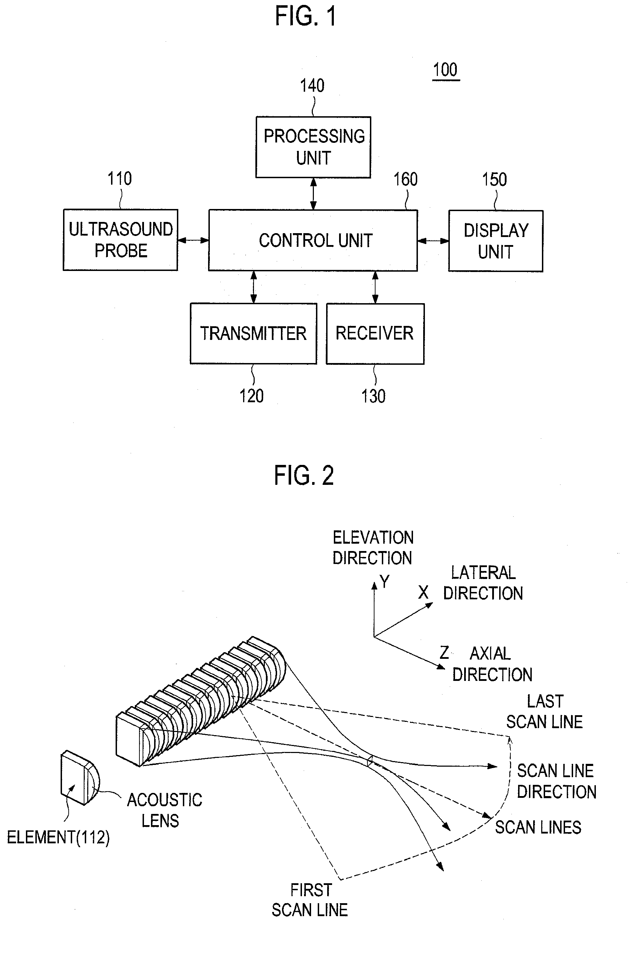

FIG. 1 is a block diagram showing an illustrative embodiment of an ultrasound system. The ultrasound system 100 may include an ultrasound probe 110 containing a plurality of transducer elements 112 for reciprocal conversion between ultrasound signals and electrical signals, as illustrated in FIG. 2. The plurality of transducer elements 112 may transmit an ultrasound beam to the target object along an axial direction and receive echo signals reflected from the target object to thereby output electrical receive signals. The receive signals may contain anatomical information of the target object in axial, lateral and elevation directions, which are necessary for ultrasound imaging.

The ultrasound system 100 may further include a transmitter 120 that may generate transmit (Tx) pulses and apply delays to the Tx pulses to form a Tx pattern thereof.

The delayed Tx pulses are transferred to the transducer elements through channels so that the ultrasound beam may be focused on focal points. The transmitter 120 may generate the Tx pulses at a transmit (Tx) frequency in synchronization with a transmit (Tx) control clock. The transmitter 120 may be further operable to control power of the Tx pulses. In one embodiment, by way of non-limiting example, the power control of the Tx pulses may be performed through the pulse width modulation (PWM).

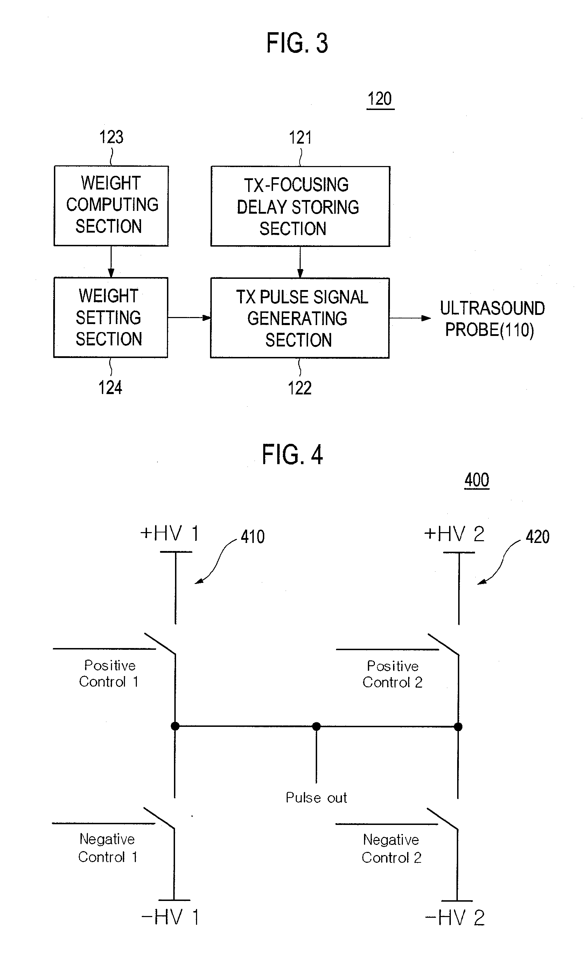

FIG. 3 is a block diagram showing an illustrative embodiment of the transmitter 120. Referring to FIG. 3, the transmitter 120 may include a Tx-focusing delay storing section 121 that may store delay patterns necessary for compensating delivery times of the ultrasound transmit beam from the respective transducer elements to focal points. The delay patterns may be determined by considering distances between the respective transducer elements and the focal points.

The transmitter 120 may further include a Tx pulse signal generating section 122 having a plurality of pulse generators, which are connected to the respective channels, to generate Tx pulses under the control of pulser control signals. The Tx pulse signal generating section 122 may be further operable to apply delays to the Tx pulses according to the delay patterns stored in the Tx-focusing delay storing section 121 to thereby output delayed Tx pulses. In one embodiment, each of the pulse generators may include multiple pulsers operating in response to the pulser control signal. For example, each of the pulse generators 400 may include dual pulsers consisting of a first pulser 410 and a second pulser 420, as shown in FIG. 4. Power supply voltages HV1 and HV2, which are denoted in FIG. 4, may represent different voltage levels. For example, HV1 may be twice higher than HV2. The first and second pulsers 410 and 420 may operate under the control of the pulser control signal Positive Control 1, Negative Control 1, Positive Control 2 and Negative Control 2.

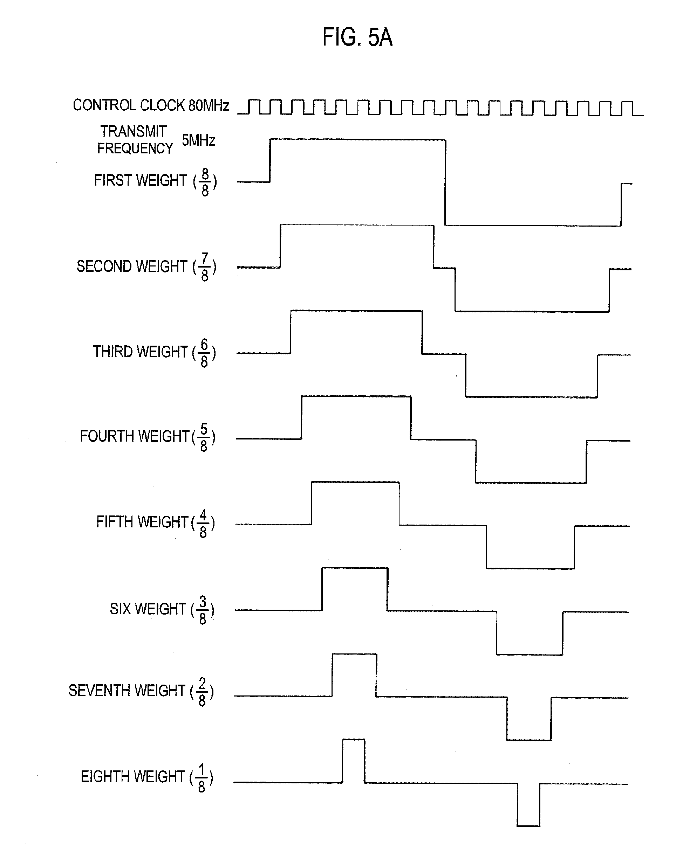

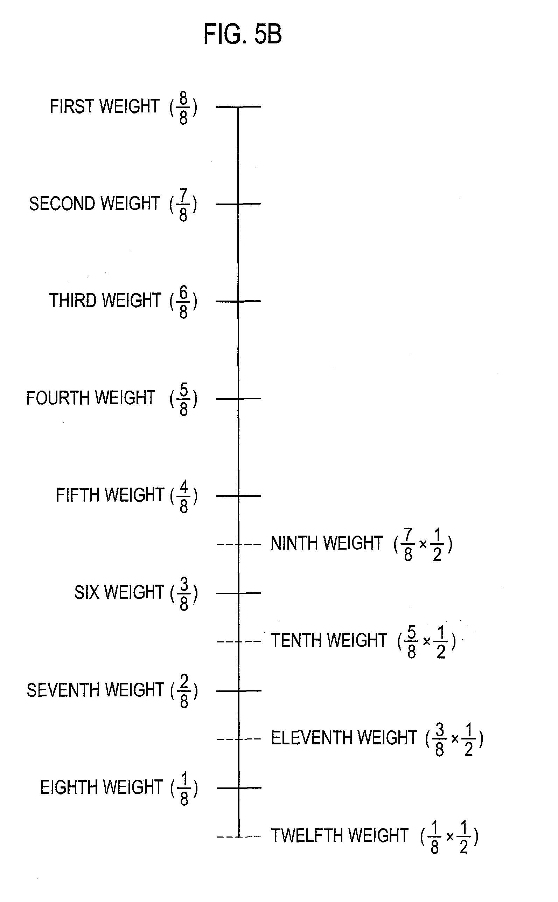

The transmitter 120 may further include a weight computing section 123 that may compute weights through the PWM of the pulser control signal for power control of the Tx pulses according to the Tx control clock, Tx frequency and the number of pulsers. For example, assuming that the Tx control clock is 80 MHz, Tx frequency is 5 MHz and the number of pulsers is 1 (i.e., single pulser), the maximum pulse duration of the pulser control signal may be 8 clocks and the minimum pulse duration of the pulser control signal may be 1 clock. Thus, the weight computing section 123 may compute 8 weights (e.g., first to eighth weights), as illustrated in FIG. 5A. In one embodiment, if the Tx control clock is 80 MHz, Tx frequency is 5 MHZ, the number of pulses is 2 (i.e., dual pulsers), and the power supply voltage HV1 is twice higher than the power supply voltage HV2, ninth to twelfth weights that may be obtained by controlling the second pulser 420 in addition to the first to eight weights, which may be obtained by controlling the first pulser 410, as illustrated in FIG. 5B. Thus, higher resolution of the weights can be obtained by using the multiple pulsers.

Although the above embodiment has been described that the first to eighth weights are computed for the first pulser 410 and the ninth to twelfth weight are computed for the second pulser 420, the weight computation is not limited thereto. In one embodiment, the first to fourth weights may be computed for the first pulser 410 and the fifth to twelfth weights may be computed for the second pulser 420.

Also, although the above embodiment has been described that the weights are computed for the dual pulsers, the weight computation is not limited thereto. In one embodiment, the weight computation may be carried out for a number of pulsers more than two pulsers.

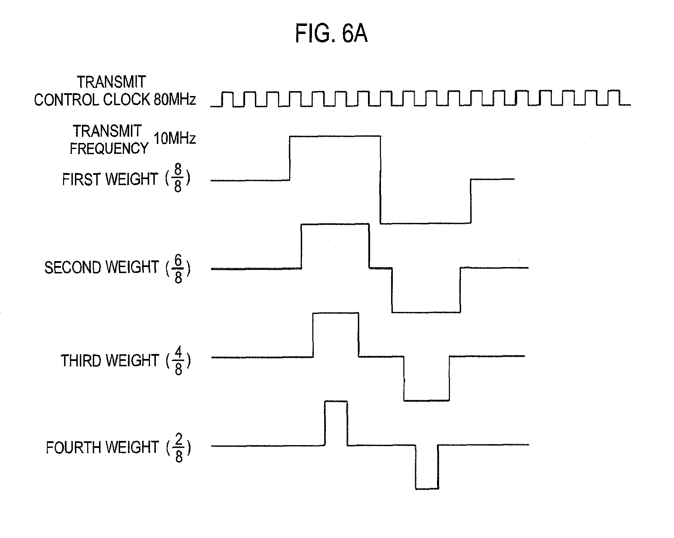

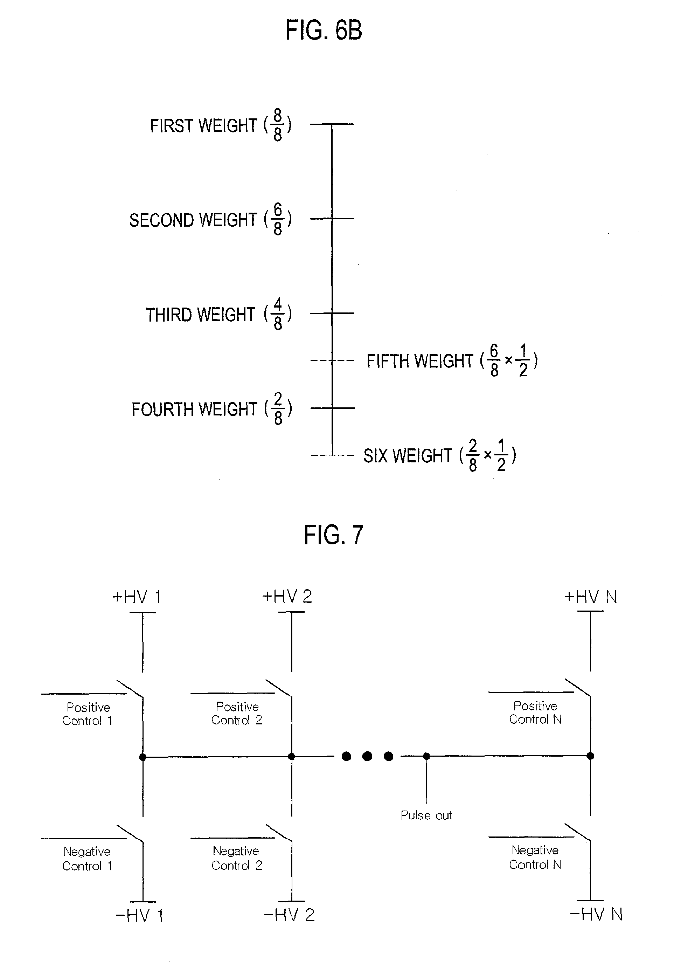

In another embodiment, if the Tx control clock is 80 MHz, the tx frequency is 10 MHz and the number of the pulsers included in each pulse generator is 1 (i.e., single pulser), the maximum pulse duration of the pulser control signal may be 4 clocks and the minimum pulse duration of the pulser control signal may be 1 clock. Thus, 4 weights (e.g., first to fourth weights) may computed, as illustrated in FIG. 6A. In one embodiment, if the Tx control clock is 80 MHz, the Tx frequency is 10 MHz, the number of the pulsers included in each pulse generator is 2 (i.e., dual pulsers), and the power supply voltage HV1 is twice higher than the power supply voltage HV2, fifth and sixth weights that may be obtained by controlling the second pulser 420 in addition to the first to fourth weights, which may be obtained by controlling the first pulser 410, as illustrated in FIG. 6B.

Although the above embodiment has been described that the first to fourth weights are computed for the first pulser 410 and the fifth and sixth weights are computed for the second pulser 420, the weight computation is not limited thereto. In one embodiment, the first and second weights may be computed for the first pulser 410 and the third to sixth weights may be computed for the second pulser 420.

The transmitter 120 may further include a weight setting section 124 that may set the pulser control signals with the computed weights and the pulser control signals are applied to the Tx pulsers to control the power thereof for apodization.

The ultrasound system 100 may further include a receiver 130 that may perform analog-to-digital conversion upon the electric receive signals outputted from the ultrasound probe 110 to thereby output digital receive signals. The receiver 130 may be further operable to apply delays to digital receive signals in consideration of distances between the focal points and the respective transducer elements. Further, the receive unit 130 may be operable to sum the delay digital signals to thereby output a receive-focused beam.

The ultrasound system 100 may further include a processing unit 140 that may be operable to form an ultrasound image based on the receive-focused beam. The ultrasound image is displayed on the display unit 150. The ultrasound system 100 may further include a control unit 160 that may be operable to control the transmission and reception of the ultrasound signals. The ultrasound system 100 may be further operable to control entire operations of elements of the ultrasound system 100.

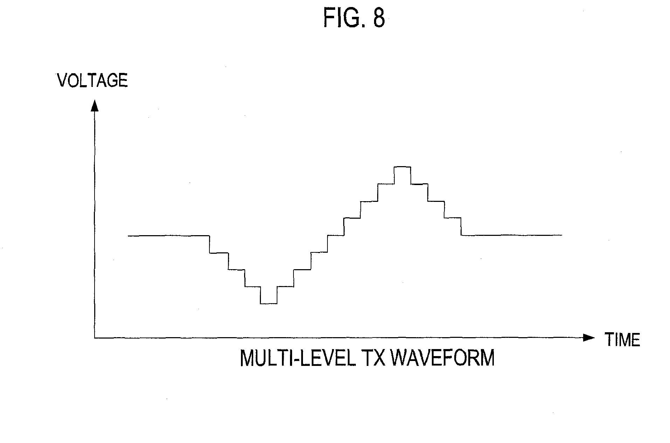

Although the above embodiment has been described that the dual pulsers are adopted to increase resolution of the weights for power control of the Tx pulses, the number of the pulsers may not be limited thereto. More than two pulsers may be adopted in each pulse generator to control the power of the Tx pulses, as illustrated in FIG. 7. In such a case, a multi-level transmit waveform may be obtained as shown in FIG. 8.

Although embodiments have been described with reference to a number of illustrative embodiments thereof, it should be understood that numerous other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasound system, comprising:
an ultrasound probe containing a plurality of transducer elements for converting between electrical and ultrasound signals; and
a transmitter including a plurality of pulse generators each connected to the respective transducer element through a channel to generate a transmit (Tx) pulse at a transmit (Tx) frequency in synchronization with a Tx control clock, each of the pulse generators having a plurality of pulsers operating at different voltages in response to pulser control signals.

2. The ultrasound system of Claim 1, wherein the transmitter includes:
a weight computing section configured to compute weights to be applied to the pulse generators according to the Tx control clock, Tx frequency and the number of pulsers included in each of the pulse generators; and
a weight setting section configured to set the computed weights to the respective pulse generators.

3. The ultrasound system of Claim 2, wherein the weights are computed through pulse width modulation of the pulser control signal.

4. The ultrasound system of Claim 1, wherein the transmitter is configured to generate the Tx pulses having a multi-level Tx waveform.
